# FASCICULE DE BREVET EUROPEEN

(11) **EP 1 827 211 B1**
(45) Date de publication et mention de la délivrance du brevet: **01.02.2012**
(21) Numéro de dépôt: 05814998.0
(22) Date de dépôt: 03.11.2005
(51) Int. Cl.: A61B 5/00, A61M 5/142, A61M 5/172

(54) **SYSTEME MEDICAL D'INJECTION COMPORTANT UNE UNITE IMPLANTABLE DANS UN CORPS VIVANT**
MEDIZINISCHES INJEKTIONSSYSTEM MIT IN EINEN LEBENDEN KÖRPER IMPLANTIERBAREM GERÄT
INJECTION MEDICAL SYSTEM COMPRISING A UNIT IMPLANTABLE INTO A LIVING BODY

(30) Priorité: 03.11.2004 FR 0411730
(43) Date de publication de la demande: 05.09.2007
(73) Titulaire: B.Braun Medical SAS, 92100 Boulogne-Billancourt (FR)
(72) Inventeur: BEAU, Jacques, F-91100 Villabe (FR); CHEVILLON, Gérard, F-92120 Montrouge (FR); LEVI, Francis, F-94800 Villejuif (FR); NADAL, Guy, F-86000 Poitiers (FR)
(74) Mandataire: Mahler, Peter
(86) Numéro de dépôt international: PCT/FR2005/002733
(87) Numéro de publication internationale: WO 2006/048554

(56) Documents cités:
- EP-A- 0 672 427
- US-A- 4 871 351
- US-A- 5 623 432
- US-A1- 2002 087 114
- US-A1- 2002 143 371

## Description

La présente invention concerne une unité implantable dans un corps vivant, un système médical d'injection destiné à l'optimisation d'une thérapie, et un procédé de traitement thérapeutique d'un patient humain ou animal, avantageusement fondé sur les rythmes biologiques.

Bien qu'elle s'applique à d'autres traitements, destinés par exemple à la thérapie des affections infectieuses, métaboliques, auto-immunes, toxiques, traumatiques, etc., on décrit l'invention dans son application à la thérapie des cancers.

La plupart des cancéreux reçoivent des perfusions chroniques par l'intermédiaire d'un site d'accès veineux implanté. Ce site est particulièrement utile pour les perfusions ambulatoires de chimiothérapie continue.

On a proposé une approche chronothérapeutique des cancers afin d'une part de réduire la toxicité des molécules et d'autre part d'en augmenter l'efficacité. La chimiothérapie est ainsi effectuée par perfusion chronomodulée avec des horaires déterminés suivant un schéma d'administration chronothérapeutique.

Cette thérapie repose sur l'étude du rythme circadien et de ses composantes (ce qu'on appelle parfois horloges biologiques). On a en effet démontré par exemple que les anti-inflammatoires non stéroïdiens étaient deux fois moins toxiques pour l'estomac lorsqu'ils étaient pris le soir plutôt que le matin. On a aussi démontré que la toxicité rénale de la gentamycine était plus faible lorsque cet antibiotique était pris par une perfusion en milieu de journée plutôt que la nuit.

Le rythme circadien comprend diverses composantes, dont des composantes ultradiennes. Ces dernières sont, pour les cas classiques et non pathologiques, en général d'influence négligeable. Cependant, pour des cas particuliers, par exemple sous l'effet d'une administration médicamenteuse, au cours des prémices d'un risque pathologique ou sous l'action d'un traitement ou d'un événement environnemental, ces composantes ultradiennes sont fondamentalement informatives et permettent une prise de décision et une possibilité d'action précoce.

Le rythme circadien est caractérisé par des fréquences correspondant à des périodes de 24 h et ses harmoniques : 12 h, 8 h, 6 h, 4,8 h, 4 h, 3,43 h, 3 h, etc. Les composantes harmoniques d'ordre élevé de ce rythme décroissent en énergie (et donc en utilité pour définir la structure temporelle). De ce fait, il ne semble pas utile de relever les informations temporelles pour des périodes inférieures à 1 h.

Cependant, plusieurs facteurs peuvent perturber le système circadien sur lequel repose la chronothérapie : des variations individuelles, la pathologie et la thérapie elle-même. En conséquence, pour un patient donné, il peut exister une différence entre l'heure générale et celle de l'horloge interne du sujet.

On s'est déjà rendu compte que la température interne profonde, qui était régulée par le système circadien, constituait un excellent et robuste marqueur de l'horloge interne d'un patient. Sa mesure donne donc un repère précis de la phase individuelle du rythme circadien et permet ainsi une optimisation du traitement par individualisation des moments d'administration.

On a donc déjà tenté d'enregistrer en continu la température corporelle humaine profonde. Cependant, on ne connaît pas de dispositif permettant un tel enregistrement hors de l'hôpital, car seule la mesure de la température rectale donne des résultats suffisamment probants, et celle-ci ne peut être relevée de façon continue chez des malades pendant des durées prolongées.

On a donc suggéré d'utiliser un détecteur associé à une chambre implantable destinée à l'exécution de perfusions. Le détecteur de température est destiné à être implanté dans le tissu sous-cutané profond, au contact des muscles, et à être isolé des influences thermiques extérieures.

Plus précisément, on a envisagé d'associer à une chambre implantable un détecteur de température et un émetteur transmettant à intervalles réguliers la valeur de la température mesurée à un récepteur d'un appareil porté à l'extérieur du corps du patient. Les signaux reçus par le récepteur sont destinés à être enregistrés dans l'appareil porté par le patient puis analysés par le personnel médical, à qui est destiné l'appareil.

Un tel système a pour objet la connaissance de la phase du rythme circadien et de sa stabilité, pour la définition du traitement.

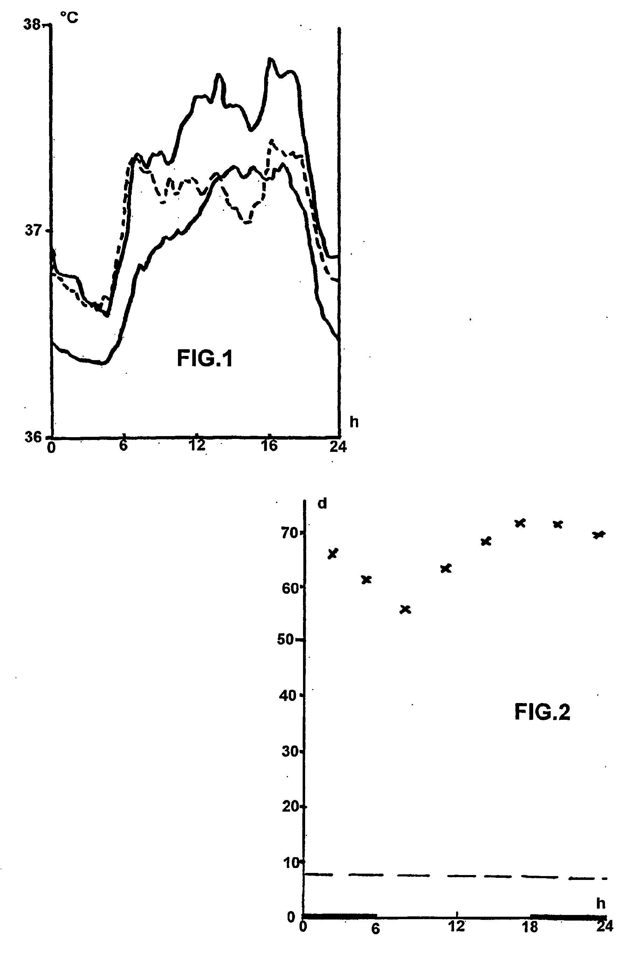

De façon annexe, on a considéré que ce système pouvait aussi permettre une détection précoce du début d'une infection ou de rythmes ultradiens. En effet, comme l'indique la figure 1 qui est un graphique représentant les résultats d'une étude réalisée par mesure de températures rectales, une simple laryngite provoque une modification significative (courbe en trait interrompu) de la température qui évolue normalement dans la plage comprise entre les deux courbes en trait plein (moyenne sur huit jours) avec une amplitude en général de l'ordre de 1 ± 0,5 °C. On conçoit facilement l'intérêt d'une alerte lancée dès la détection d'une telle infection.

Le système précité a été conçu, mais non encore réalisé, pour permettre une meilleure connaissance du patient et une meilleure adaptation du traitement à celui-ci. Cependant, cette adaptation n'est que globale dans la mesure où les résultats obtenus correspondent à des instructions générales, par exemple à un rythme constant de transmission de la température mesurée, quels que soient la période de la journée et l'état du patient, si bien que les résultats obtenus ne sont que globaux.

Il serait avantageux d'obtenir les résultats non seulement afin qu'ils puissent être utilisés rapidement, et même très rapidement en cas d'incident, mais aussi d'une manière qui s'adapte à l'état du patient, notamment aux variations qui peuvent se présenter, à l'évolution de la pathologie et aux conséquences de la thérapie.

On sait en effet qu'un affinement du traitement d'un cancéreux, par exemple par perfusion continue ou par administrations quotidiennes répétées au lieu d'une seule permet une augmentation considérable de la tolérance ou de l'efficacité du traitement. De plus, des essais effectués sur des souris ont montré qu'une injection toutes les trois heures d'une quantité modulée de façon sinusoïdale sur la journée donne des résultats meilleurs qu'une injection non modulée, et surtout qu'une injection unique de quantité plus élevée. Ainsi, la figure 2 indique que le temps de survie (en ordonnées) des souris atteint 55 à 72 jours avec modulation, alors que l'injection unique donne un temps de survie qui n'atteint pas dix jours.

L'invention a pour objet d'obtenir des résultats précis et significatifs obtenus par mesure d'au moins un paramètre d'un corps vivant, de préférence la température profonde du corps vivant, à une fréquence adaptée à l'état de ce corps, avec un système aussi simple et robuste que possible, mettant en oeuvre les technologies déjà éprouvées pour l'échange de données entre un dispositif implanté dans un corps vivant et un dispositif extérieur.

On connaît déjà de nombreux systèmes dans lesquels une unité implantable dans un corps vivant comprend un détecteur d'au moins un paramètre, et un émetteur de données associé à une antenne et qui exécute une émission de données représentatives du paramètre à une fréquence d'émission, les données étant destinées à un récepteur extérieur.

Ainsi, le document US-4 871 351 décrit un système qui comprend un ensemble implantable possédant une pompe d'administration de médicaments commandée par échange d'informations entre l'ensemble implantable et un ensemble extérieur. Ce système transmet des données relatives au fonctionnement de l'ensemble implantable, mais ne suggère pas la mesure régulière d'un paramètre du corps vivant dans lequel est implanté l'ensemble.

De même, les documents FR-2 792 841, US2002/042596, US2002/156462 et EP-672 427 décrivent des systèmes comportant une unité implantable, assurant parfois une mesure, mais toujours d'un paramètre de fonctionnement de l'appareil implanté et non du corps vivant. En outre, aucun d'eux ne suggère la modulation de la fréquence de mesure en fonction des résultats des mesures. Seule la mise en veille est suggérée, pour des raisons d'économie d'énergie, et non en fonction des résultats.

L'invention a pour objet de tirer parti de la technologie connue de communication de données entre un ensemble implantable et un ensemble extérieur pour affiner la connaissance de l'état actuel du patient, et donc pour accroître l'efficacité du traitement du patient ou de sa surveillance, par adaptation des conditions de mesure soit directement, soit avec traitement local des données, grâce à la modulation des conditions de mesure.

Plues précisément, l'invention concerne un système médical d'injection tel que défini dans la revendication 1. Un document qui divulgue les caractéristiques techniques du préambule est le US 2002/0087114.

Dans un mode de réalisation avantageux, l'unité implantable comporte en outre une mémoire contenant des données représentatives d'au moins un cycle de variations des données, ce cycle de variations ayant avantageusement une durée de vingt-quatre heures.

Dans un mode de réalisation avantageux, le dispositif de commande et de régulation comporte en outre un dispositif de création de données d'alerte destiné à émettre des données. Par exemple, les données d'alerte sont obtenues par comparaison de valeurs du signal du capteur à un seuil supérieur ou inférieur, ou par comparaison de valeurs échantillonnées à un seuil supérieur ou inférieur.

De préférence, les données d'alerte sont destinées à déclencher une opération de traitement des valeurs échantillonnées par le dispositif de commande et de régulation, par exemple en fonction de la valeur du signal du capteur ou de la valeur échantillonnée, en fonction de la variation de la valeur du signal du capteur ou de la valeur échantillonnée, en fonction de la différence entre des valeurs successives du signal du capteur ou de la valeur échantillonnée, ou en fonction du résultat de la comparaison de la valeur du signal du capteur ou de la valeur échantillonnée à une valeur mémorisée, cette valeur mémorisée ayant par exemple été déduite des données relevées 24 h auparavant ou un multiple de 24 h auparavant.

De préférence, le capteur est un capteur de la température profonde du corps vivant, bien que d'autres paramètres puissent être mesurés, tels qu'un paramètre représentatif de l'état de repos-activité, la concentration du glucose ou de l'ion calcium, d'autres paramètres liés à la pression artérielle ou au rythme cardiaque et d'autres paramètres de nature physique, chimique ou biologique, tels que le pH d'un fluide. Plusieurs détecteurs peuvent avantageusement mesurer plusieurs paramètres.

De préférence, l'échantillonneur et le dispositif de commande et de régulation, avantageusement avec le dispositif d'alerte, forment un seul dispositif électronique.

Dans un mode de réalisation avantageux, l'invention permet une interaction bidirectionnelle entre la partie implantée et la partie extérieure. En effet, il n'est guère envisageable d'implanter un système électronique capable d'effectuer par lui-même d'importants traitements de données tenant compte de nombreux paramètres, car il devrait être extrêmement élaboré, volumineux et gourmand en énergie. En conséquence, dans un système de base, seules les conditions de mesure et d'émission d'un paramètre peuvent être modifiées par le système extérieur. Dans un système plus élaboré, le dispositif implanté exécute lui-même un traitement sommaire de données, par exemple permettant la modulation de la fréquence de mesure et/ou la détermination d'une condition d'alerte.

L'invention concerne ainsi un système médical d'injection destiné à l'optimisation d'une thérapie qui comprend un ensemble extérieur, comportant un premier récepteur de données, et un ensemble d'injection implantable dans un corps vivant, et comprenant d'une part un dispositif d'injection et d'autre part une unité implantable telle que définie dans les paragraphes précédents.

De préférence, l'ensemble extérieur comprend un second émetteur de données et un dispositif de traitement de données, et l'ensemble implantable d'injection comprend un second récepteur de données destiné à recevoir des données d'instruction du second émetteur, et un dispositif de commande et de régulation destiné, en fonction des instructions reçues par le second récepteur, à déterminer au moins une condition choisie parmi les conditions de mesure du paramètre du corps vivant par le détecteur et les conditions d'émission, par le premier émetteur, des données correspondant à une valeur mesurée par le détecteur.

Dans un mode de réalisation, l'ensemble extérieur comprend au moins deux parties, une première partie comportant au moins un dispositif d'enregistrement de données provenant de l'ensemble implantable, et une seconde partie comportant le dispositif de traitement des données, la seconde partie étant destinée à recevoir sur commande des données du dispositif d'enregistrement de données.

De préférence, le dispositif de traitement de l'ensemble extérieur est destiné à adapter les données d'instruction d'une part aux données reçues depuis le premier émetteur et d'autre part à d'autres informations relatives au corps vivant dans lequel est implanté l'ensemble implantable.

Dans un mode de réalisation, une condition déterminée par le dispositif de commande et de régulation comprend une condition au moins liée à la fréquence à laquelle le détecteur mesure le paramètre du corps vivant.

Dans un exemple de réalisation, une condition déterminée par le dispositif de commande et de régulation comprend une condition au moins liée à la période déterminée d'exécution de l'émission des données relatives au paramètre du corps vivant par le premier émetteur.

De préférence, le dispositif de commande et de régulation de l'ensemble implantable est en outre destiné à exécuter un traitement de données.

De préférence, le traitement de données exécuté par le dispositif de commande et de régulation comprend un traitement de compression des données à émettre par le premier émetteur.

Dans un mode de réalisation avantageux, le système comporte en outre un dispositif d'alerte, et le dispositif de commande et de régulation de l'ensemble implantable déclenche, directement ou non, le dispositif d'alerte lorsqu'une condition liée au paramètre mesuré par le détecteur est remplie. Par exemple, le dispositif d'alerte est tenu par la partie extérieure portée par le patient qui peut être immédiatement avisé, par exemple par un message détaillé affiché sur cette partie. Dans un exemple, la condition liée au paramètre mesuré est le dépassement d'au moins une valeur limite inférieure ou supérieure du paramètre.

De préférence, l'ensemble implantable d'injection est une chambre implantable en sous-cutané profond destinée à la diffusion ou au recueil de liquide, ou une pompe implantable d'injection de liquide.

De préférence, l'ensemble implantable d'injection comporte en outre une alimentation électrique indépendante.

Un procédé de traitement d'une affection d'un patient humain ou animal comprend la détermination du rythme circadien du patient affecté par l'affection par mesure de sa température profonde, et l'administration au patient d'au moins une composition efficace pour le traitement de l'affection du patient à au moins un moment de la journée déterminé d'après le rythme circadien ; l'étape de détermination du rythme circadien du patient comprend la mesure de la température profonde du patient à une fréquence de mesure déterminée par une unité implantée, l'évaluation de l'état du patient par comparaison du rythme circadien ainsi déterminé en continu à un rythme circadien de référence du patient dans l'unité implantée, et, suivant l'état ainsi déterminé, l'exécution d'au moins une opération choisie parmi la modification de la fréquence de mesure, la transmission de données spécifiques d'alerte, et la modification de l'administration d'une composante efficace.

De préférence, le procédé comprend l'obtention de données spécifiques d'alerte par comparaison de valeurs du signal mesuré ou de valeurs échantillonnées à un seuil supérieur ou inférieur.

De préférence, l'étape d'exécution d'au moins une opération comprend l'exécution d'une opération de traitement de valeurs échantillonnées par le dispositif de commande et de régulation en fonction de la valeur du signal du capteur ou de la valeur échantillonnée, de la variation de la valeur du signal mesuré ou de la valeur échantillonnée, de la différence entre des valeurs successives du signal mesuré ou de la valeur échantillonnée, ou du résultat de la comparaison de la valeur du signal mesuré ou de la valeur échantillonnée à une valeur mémorisée.

Le procédé est par exemple destiné au traitement non seulement des cancers, mais aussi à celui des affections infectieuses, métaboliques, auto-immunes, toxiques, traumatiques, etc.

D'autres caractéristiques et avantages de l'invention seront mieux compris à la lecture de la description qui va suivre d'exemples de réalisation, faite en référence aux dessins annexés sur lesquels :
les figures 1 et 2 ont déjà été décrites ;
la figure 3 est un diagramme synoptique représentant les principaux éléments d'un dispositif médical d'optimisation de thérapie selon l'invention ;
la figure 4 est un diagramme synoptique représentant les principaux éléments d'un dispositif médical bidirectionnel d'optimisation de thérapie selon l'invention ; et
la figure 5 représente une variante de la partie extérieure du dispositif de la figure 4.

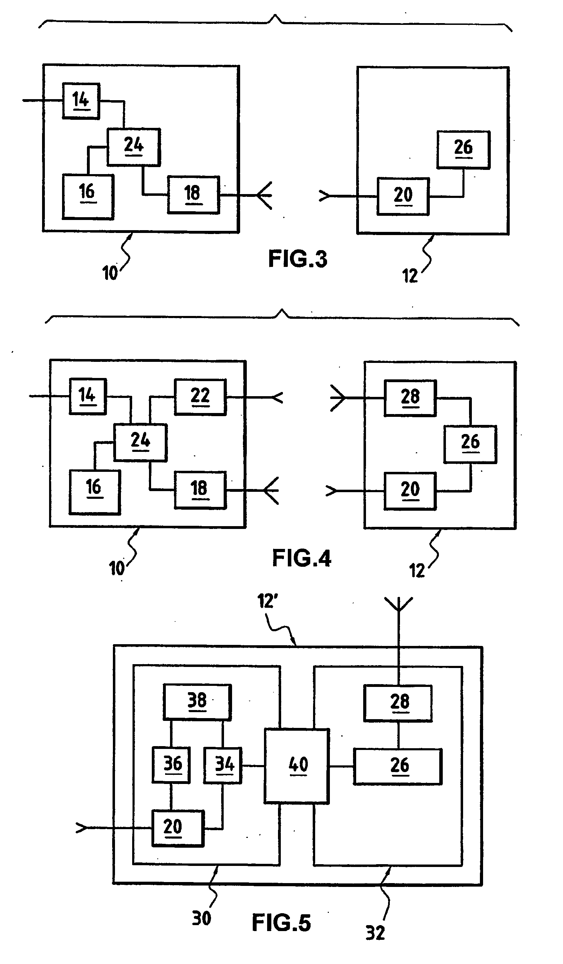

La figure 3 représente un dispositif médical qui comporte un ensemble implantable 10 et un ensemble extérieur 12.

Dans ce système de type unidirectionnel, l'ensemble implantable 10 est une chambre implantable munie d'un module électronique comprenant un détecteur 14 de température, comprenant un capteur et un échantillonneur, une alimentation 16, en général une pile électrique, et un émetteur 18 qui est destiné à émettre des données correspondant à la valeur de la température obtenue par le détecteur 14. Un dispositif 24 de commande et de régulation assure le fonctionnement de l'ensemble du système. Ces données sont reçues par un récepteur 20 d'un ensemble extérieur 12 qui comporte aussi un circuit 26 de traitement, enregistrées, puis utilisées pour l'évaluation des rythmes circadiens du patient et de leur évolution.

La figure 4 représente un système de type bidirectionnel qui comporte aussi un ensemble implantable 10 et un ensemble extérieur 12. En plus des éléments décrits en référence à la figure 3, l'ensemble implantable 10 comporte un récepteur 22, et l'ensemble extérieur comporte un second émetteur de données 28 capable d'émettre des signaux destinés au récepteur 22.

Les signaux reçus par le récepteur 22 sont traités par le circuit 24 de commande et de régulation afin que celui-ci effectue ou commande diverses opérations : variation du rythme de détection du paramètre ou des paramètres, variation du rythme d'émission des données par l'émetteur 18, détermination de conditions d'urgence nécessitant le déclenchement d'une alerte, etc.

Dans un exemple de réalisation, l'ensemble implantable 10 est formé par une chambre implantable double munie de réservoirs de dimensions réduites pour que le circuit électronique et l'antenne puissent être logés. Le volume total du circuit électronique de l'ensemble implantable est inférieur à 1 cm³. Lorsque le paramètre mesuré est la température, le détecteur donne une résolution de 0,1 °C et une précision absolue de 0,2 °C, dans la plage de températures comprises entre 35 et 42 °C. L'échantillonnage de la température peut être exécuté toutes les minutes et même plus fréquemment en cas d'urgence, ou toutes les dix minutes ou même toutes les heures ou plus en période de repos.

L'antenne de la chambre implantable peut n'occuper qu'un faible volume. Ainsi, elle peut être constituée d'une simple boucle disposée par exemple d'un côté de la chambre, lorsque les réservoirs sont métalliques. Comme cette disposition peut présenter des contraintes d'émission, notamment de directivité des signaux émis, il est possible d'utiliser d'autre réalisations.

Dans une première variante, le réservoir ou les réservoirs ne sont pas conducteurs : ils sont par exemple formés de céramique, afin qu'une boucle d'antenne puisse entourer pratiquement toute la chambre. Dans une seconde variante, un réservoir au moins, de forme circulaire, peut constituer lui-même une antenne, par exemple par dépôt d'un dessin d'antenne sur un réservoir d'un matériau non conducteur. Dans une troisième variante, une longue antenne efficace peut être disposée le long du cathéter auquel est connectée la chambre implantable.

La figure 5 représente une variante de l'ensemble extérieur 12. L'ensemble extérieur 12' comprend deux parties séparables 30 et 32. La partie 30 est destinée à être transportée par le patient, alors que la partie 32 est destinée à être située dans des locaux médicaux.

La partie 30 transportable par le patient comprend non seulement le récepteur de données 20, mais aussi un dispositif d'enregistrement de données 34, un dispositif de commande 36 et, de préférence, une alerte 38. La partie 32 destinée à être située dans des locaux médicaux comprend le circuit de traitement de données 26 et éventuellement le second émetteur 28, bien que celui-ci puisse aussi se trouver dans la partie transportable 30.

La référence 40 désigne un dispositif de connexion temporaire de la partie 30 transportable par le patient et de la partie 32 destinée à être située dans des locaux médicaux. Ce dispositif de connexion est bidirectionnel. Il peut s'agir de connecteurs électriques. Cependant, il peut aussi s'agir d'une liaison sans fil, réalisée entre des émetteurs-récepteurs disposés l'un dans la partie transportable 30 et l'autre dans la partie 32 destinée à être située dans des locaux médicaux.

La référence 38 désigne un dispositif d'alerte, par exemple un ronfleur, un vibreur ou une liaison à hautes fréquences vers un centre de gestion. Ce dispositif est de préférence incorporé à la partie transportable par le patient. De cette manière, lorsque le circuit 24 de commande et de régulation de la partie implantable 10 détecte une condition d'alerte, par exemple une variation excessivement rapide ou anormale de l'évolution d'au moins un paramètre mesuré, par comparaison à des seuils de valeurs ou de variation, ou tout simplement le défaut de fonctionnement d'un élément quelconque, il commande l'émission d'un signal d'alerte par l'émetteur 18, si bien que le circuit de commande 36 de la partie transportable 30 peut déterminer cette condition d'alerte et commander l'alerte 38. L'utilisateur est de préférence avisé de la nature de l'alerte par un message présenté sur un afficheur.

Bien entendu, pour des raisons de sécurité, le circuit de commande et de régulation 24 peut commander de manière connue la transmission d'un signal indiquant que le dispositif implantable fonctionne effectivement, afin que, en l'absence de réception d'un signal quelconque, le circuit de commande 36 de la partie transportable puisse déterminer cette absence d'émission ou la perturbation de la transmission et en déduire l'existence d'un défaut de transmission. On ne décrit pas plus en détail ce système, car il est bien connu dans le domaine des dispositifs de sécurité.

La présence d'un circuit 24 de commande et de régulation permet un traitement sommaire des données du détecteur 14 destiné à réduire le nombre d'opérations les plus consommatrices d'énergie. En effet, l'alimentation 16 de la partie implantable doit pouvoir assurer le fonctionnement pendant plusieurs années, et il est donc nécessaire non seulement qu'elle ait un petit volume et donc une grande densité d'énergie, mais aussi que l'énergie consommée soit aussi réduite que possible.

L'une des fonctions essentielles du circuit 24 de commande et de régulation est de réduire au maximum le fonctionnement de l'émetteur, soit par réduction de sa durée ou de sa fréquence de fonctionnement, soit par réduction des données émises. Par exemple, la transmission d'un signal indiquant que le paramètre garde la même valeur que le signal précédent consomme moins d'énergie que la transmission d'un signal représentant la valeur elle-même. Un tel traitement doit évidemment être réalisé dans la partie implantable.

L'émission par la partie implantable a une périodicité longue si le paramètre ne varie pas significativement, et plus courte dans le cas contraire, avec éventuellement des "bips" intermédiaires indiquant que le système n'est pas en panne.

La modulation des conditions de mesure pose cependant certains problèmes. D'après le théorème de Nyquist, l'échantillonnage à une certaine période permet d'accéder à des informations de période double (correspondant à la fréquence de Nyquist). Si des composantes du signal échantillonné ont une énergie importante au delà de la fréquence de Nyquist (deux fois la fréquence d'échantillonnage), le relevé du signal échantillonné est fondamentalement erroné, et on peut observer un effet stroboscopique par exemple.

Il faut donc que la fréquence d'échantillonnage soit suffisante lorsque le rythme d'évolution du paramètre mesuré (la température dans le cas considéré) est perturbé de manière rapide, et que la vitesse de prise des échantillons croisse. En pratique, si l'on échantillonne à une fréquence suffisamment élevée, le signal est convenablement échantillonné, et on peut en déduire que cette fréquence est trop élevée et donc réduire le nombre d'échantillons utiles à enregistrer ou à transmettre. Si le signal est échantillonné à une fréquence insuffisante, on ne peut savoir s'il est nécessaire d'augmenter cette fréquence puisque le signal est échantillonné de façon erronée.

Des analyses plus élaborées peuvent aussi être effectuées comme par exemple le calcul du spectre par transformée de Fourier ou des filtrages adaptés afin de déterminer la période optimale de conservation des données.

En résumé, si l'on échantillonne trop vite par rapport à une fréquence jugée optimale, le signal est convenablement capté, mais la quantité d'information retenue et l'énergie dépensée sont surabondantes ; si l'on n'échantillonne pas assez vite par rapport à une fréquence jugée optimale, le signal n'est pas convenablement capté et ne peut pas être récupéré. La fréquence jugée optimale varie cependant avec l'agression que subit le patient, la régularité ou l'irrégularité de la perturbation que provoque cette agression, etc. de sorte que la modulation des conditions de mesure dépend du patient lui-même.

Ainsi, la modulation des conditions de mesure, et donc le traitement de données effectué au moins en partie dans le circuit 24 de commande et de régulation, a une incidence primordiale sur la qualité des résultats obtenus et sur les ressources en énergie nécessaires dans l'ensemble implanté.

On a considéré un système dans lequel le dispositif d'alerte est incorporé à la partie transportable de l'ensemble extérieur. Cependant, il est aussi possible d'exécuter la fonction d'alerte dans la partie 32 située dans des locaux médicaux. Dans ce cas, il est avantageux que la liaison 40 entre les parties 30 et 32 de l'ensemble extérieur soit réalisée sans fil, par exemple par un téléphone portable ou une autre liaison à hautes fréquences.

En effet, le dispositif de traitement 26 de l'ensemble extérieur 32 peut être un dispositif de traitement commun qui gère des données relatives à de nombreux patients et qui permet la détermination de conditions qui ne pourraient pas être déterminées à partir d'un seul patient.

Un tel type de liaison permet l'adaptation du traitement médical de chaque patient non seulement aux données individuelles du patient, mais aussi à d'autres données concernant par exemple des groupes de patients, et permet de faire bénéficier au plus tôt chaque patient des connaissances tirées de l'évolution de la maladie de plusieurs patients ou d'un groupe de patients simultanément.

Ainsi, les prescriptions décisionnelles du personnel médical peuvent tenir compte des connaissances acquises à partir d'un ensemble de patients.

En résumé, l'invention concerne un système médical qui permet de connaître des caractéristiques intrinsèques et évolutives du rythme circadien du patient concerné, et d'adapter le traitement d'après ces caractéristiques.

L'application de ce système médical à des traitements thérapeutiques d'affections, notamment de cancers; est envisagé.

Bien entendu, diverses modifications peuvent être apportées par l'homme de l'art aux systèmes qui viennent d'être décrits uniquement à titre d'exemple non limitatif sans sortir du cadre de l'invention.

## Revendications

1. Système médical d'injection destiné à l'optimisation d'une thérapie comprenant :
un ensemble extérieur (12), comprenant un premier récepteur de données (20), et
un ensemble d'injection (10) implantable dans un corps vivant, comprenant d'une part un dispositif d'injection et d'autre part une unité implantable (10) dans un corps vivant, l'unité implantable comprenant:
- un détecteur (14) d'au moins un paramètre du corps vivant comportant un capteur d'au moins un signal représentatif d'au moins un paramètre du corps vivant, et un échantillonneur relié au capteur et destiné à échantillonner la valeur du signal du capteur à une fréquence de mesure, et
- un émetteur (18) de données, associé à une antenne et qui exécute une émission de données représentatives du paramètre au moins à une fréquence d'émission, les données étant destinées à un récepteur extérieur,
**caractérisé en ce que**
- l'unité implantable (10) comporte un dispositif de commande et de régulation (24) destiné à régler la fréquence de mesure, et la fréquence d'émission de données en fonction de la fréquence de mesure,
la fréquence de mesure et la fréquence d'émisson croissant avec la vitesse de variation du paramètre mésuré.

2. Système selon la revendication 1, **caractérisé en ce que** l'unité implantable (10) comporte en outre une mémoire contenant des données représentatives d'au moins un cycle de variations des données.

3. Système selon l'une des revendications 1 ou 2, **caractérisé en ce que** le dispositif de commande et de régulation (24) de l'unité implantable comporte en outre un dispositif de création de données d'alerte destiné à émettre des données.

4. Système selon l'une quelconque des revendications précédentes, **caractérisé en ce que** le capteur est un capteur de la température profonde du corps vivant.

5. Système selon l'une des revendications 3 ou 4, **caractérisé en ce que** l'échantillonneur et le dispositif de commande et de régulation (24) de l'unité implantable forment un seul dispositif électronique, en coopération avec le dispositif d'alerte.

6. Système selon l'une quelconque des revendications 1 à 5, **caractérisé en ce que** le l'ensemble extérieur (12, 12') comprend un second émetteur (28) de données et un dispositif (26) de traitement de données, et l'ensemble implantable d'injection (10) comprend un second récepteur (22) de données destiné à recevoir des données d'instruction du second émetteur (28), et un dispositif de commande et de régulation (24) destiné, en fonction des instructions reçues par le second récepteur (22), à déterminer au moins une condition choisie parmi les conditions de mesure du paramètre du corps vivant par le détecteur (14) et les conditions d'émission, par le premier émetteur (18), des données correspondant a une valeur mesurée par le détecteur (14).

7. Système selon l'une quelconque des revendications 1 à 6, **caractérisé en ce que** l'ensemble extérieur (12') comprend au moins deux parties, une première partie (30) comportant au moins un dispositif (34) d'enregistrement de données provenant de l'ensemble implantable, et une seconde partie (32) comportant le dispositif (26) de traitement des données, la seconde partie (32) étant destinée a recevoir sur commande des données du dispositif (34) d'enregistrement de données.

8. Système selon l'une quelconque des revendications 1 à 7, **caractérisé en ce que** le dispositif (26) de traitement de l'ensemble extérieur (32) est destiné à adapter les données d'instruction d'une part aux données reçues depuis le premier émetteur (18) et d'autre part à d'autres informations relatives au corps vivant dans lequel est implante l'ensemble implantable (10).

9. Système selon l'une quelconque des revendications 1 à 8, **caractérisé en ce qu'**une condition déterminée par le dispositif de commande et de régulation (24) comprend une condition au moins liée à la fréquence à laquelle le détecteur (14) mesure le paramètre du corps vivant.

10. Système selon l'une quelconque des revendications 1 à 9, **caractérisé en ce qu'**une condition déterminée par le dispositif de commande et de régulation (24) comprend une condition au moins liée à la période déterminée d'exécution de l'émission des données relatives à un paramètre du corps vivant par le premier émetteur (18).

11. Système selon l'une quelconque des revendications 1 à 10, **caractérisé en ce que** le dispositif de commande et de régulation (24) de l'ensemble implantable est en outre destiné à exécuter un traitement de données.

12. Système selon la revendication 11, **caractérisé en ce que** le traitement de données exécuté par le dispositif de commande et de régulation (24) de l'ensemble implantable comprend un traitement de compression des données à émettre par le premier émetteur (18).

13. Système selon l'une quelconque des revendications 1 à 12, **caractérisé en ce qu'**il comporte en outre un dispositif d'alerte (38), et **en ce que** le dispositif de commande et de régulation (24) de l'ensemble implantable déclenche, directement ou non, le dispositif d'alerte (38) lorsqu'une condition liée au paramètre mesuré par le détecteur (14) est remplie.

14. Système selon la revendication 13, **caractérisé en ce que** la condition liée au paramètre mesuré est le dépassement d'au moins une valeur limite inférieure ou supérieure du paramètre.

## Claims

1. A medical injection system intended for the optimization of a therapy comprising:
an external assembly (12), comprising a first data receiver (20), and
an injection assembly (10) implantable into a living body, and comprising on the one hand an injection device and on the other hand a unit (10) implantable into a living body, the implantable unit comprising:
- a sensor (14) for at least one parameter of the living body, the sensor (14) including a pick-up for at least one signal representing at least one parameter of the living body, and a sampling device connected to the pick-up and intended to sample the value of the pick-up signal at a frequency of measurement, and
- a data transmitter (18), associated with an antenna and which carries out the transmission of data representing the parameter at least at a frequency of transmission, the data being intended for an external receiver,
**characterized in that**
- the implantable unit (10) includes a control and adjustment device (24) intended to adjust the frequency of measurement and the frequency of data transmission depending on the frequency of measurement,
the frequency of measurement and the frequency of transmission increasing with the rate of variation of the measured parameter.

2. System according to claim 1, **characterized in that** that the implantable unit (10) further includes a memory containing data representing at least one cycle of variations of the data.

3. System according to claims 1 or 2, **characterized in that** the control and adjustment device (24) of the implantable unit further includes a warning data creation device intended to transmit data.

4. System according to any one of the preceding claims, **characterized in that** the pick-up is a pick-up for the core temperature of the living body.

5. System according to claim 3 or 4, **characterized in that** the sampling device and the control and adjustment device (24) form a single electronic device, in co-operation with the warning device.

6. System according to any one of claims 1 to 5, **characterized in that** the external assembly (12, 12') comprises a second data transmitter (28) and a data processing device (26), and the implantable injection assembly (10) comprises a second data receiver (22) intended to receive instruction data from the second transmitter (28), and a control and adjustment device (24) intended, depending on the instructions received by the second receiver (22), to determine at least one condition selected from the conditions for measurement of the living body parameter by the sensor (14) and the conditions for transmission, by the first transmitter (18), of the data corresponding to a value measured by the sensor (14).

7. System according to any one of claims 1 to 6, **characterized in that** the external assembly (12') comprises at least two parts, a first part (30) including at least one device (34) for recording data coming from the implantable assembly, and a second part (32) including the data processing device (26), the second part (32) being intended to receive, on command, data from the data recording device (34).

8. System according to any one of claims 1 to 7, **characterized in that** the processing device (26) of the external assembly (32) is intended to adapt the instruction data on the one hand to the data received from the first transmitter (18) and on the other hand to other data relating to the living body into which the implantable assembly (10) is implanted.

9. System according to any one of claims 1 to 8, **characterized in that** a condition determined by the control and adjustment device (24) comprises a condition at least linked to the frequency with which the sensor (14) measures the living body parameter.

10. System according to any one of claims 1 to 9, **characterized in that** a condition determined by the control and adjustment device (24) comprises a condition at least linked to the specific period of execution of the transmission of data relating to a living body parameter by the first transmitter (18).

11. System according to any one of claims 1 to 10, **characterized in that** the control and adjustment device (24) of the implantable assembly is further intended to carry out data processing.

12. System according to claim 11, **characterized in that** the data processing carried out by the control and adjustment device (24) of the implantable assembly comprises a process of compression of the data to be transmitted by the first transmitter (18).

13. System according to any one of claims 1 to 12 **characterized in that**, if further includes a warning device (38), and **in that** the control and adjustment device (24) of the implantable assembly triggers, directly or otherwise, the warning device (38) when a condition linked to the parameter measured by the sensor (14) is fulfilled.

14. System according to claim 13, **characterized in that** the condition linked to the parameter measured is that of exceeding at least one lower or upper limit value of the parameter.

## Patentansprüche

1. Medizinisches Injektionssystem zur Optimierung einer Therapie umfassend:
eine externe Anordnung (12), die einen ersten Datenempfänger (20) umfasst,
und
eine in einen lebenden Körper implantierbare Injektionsanordnung (10), die einerseits eine Injektionseinrichtung und andererseits eine in einen lebenden Körper implantierbare Einheit (10) umfasst, wobei die implantierbare Einheit umfasst:
- einen Detektor (14) für mindestens ein Parameter des lebenden Körpers, der einen Sensor für mindestens ein Signal aufweist, das charakteristisch für mindestens ein Parameter des lebenden Körpers ist, und eine Probennahmevorrichtung, die mit dem Sensor verbunden und dazu bestimmt ist, Proben des Signalwerts des Sensors mit einer Messfrequenz zu nehmen, und
- einen Datensender (18), der mit einer Antenne verbunden ist, und der eine Versendung von Daten, die charakteristisch für das Parameter sind, mindestens mit einer Sendefrequenz vornimmt, und die bestimmt sind für einen äußeren Empfänger,
**dadurch gekennzeichnet, dass**
- die implantierbare Einheit (10) eine Steuer- und Regelvorrichtung (24) umfasst, die zur Regelung der Messfrequenz und der Sendefrequenz der Daten in Abhängigkeit der Messfreqeunz bestimmt ist,
wobei die Messfrequenz und die Sendefrequenz mit der Änderungsrate des gemessenen Parameters steigen.

2. System nach Anspruch 1, **dadurch gekennzeichnet, dass** die implantierbare Einheit (10) außerdem einen Speicher umfasst, der Daten, die mindestens einem Variationszyklus der Daten entsprechen, enthält.

3. System nach Anspruch 1 oder 2, **dadurch gekennzeichnet, dass** die Steuer- und Regelvorrichtung (24) der implantierbaren Einheit außerdem eine Vorrichtung zur Erzeugung von Alarmwerten umfasst, die zur Versendung der Daten bestimmt ist.

4. System nach einem der vorhergehenden Ansprüche, **dadurch gekennzeichnet, dass** der Sensor ein Sensor der Tiefentemperatur des lebenden Körpers ist.

5. System nach Anspruch 3 oder 4, **dadurch gekennzeichnet, dass** die Probennahmevorrichtung und die Steuer- und Regelvorrichtung (24) der implantierbaren Einheit eine einzige elektronische Vorrichtung darstellen, die mit der Alarmvorrichtung kooperiert.

6. System nach einem der Ansprüche 1 bis 5, **dadurch gekennzeichnet, dass** die externe Anordnung (12, 12') einen zweiten Datensender (28) und eine Datenverarbeitungsvorrichtung (26) umfasst, und die implantierbare Injektionsanordnung (10) einen zweiten Datenempfänger (22) umfasst, der dazu bestimmt ist, die Steuerdaten des zweiten Senders (28) zu empfangen, und eine Steuer- und Regelvorrichtung (24), die dazu bestimmt ist, in Abhängigkeit der vom zweiten Empfänger (22) erhaltenen Steuerbefehle mindestens eine Bedingung aus den Messwerten des Parameters des lebenden Körpers durch den Sensor (14) und den Sendebedingungen des ersten Senders (18) zu bestimmen, wobei die Daten einem durch den Detektor (14) gemessenen Messwert entsprechen.

7. System nach einem der Ansprüche 1 bis 6, **dadurch gekennzeichnet, dass** die externe Anordnung (12') mindestens zwei Teile umfasst, von denen ein erster Teil (30) mindestens eine Datenaufzeichnungsvorrichtung (34) für Daten von der implantierbaren Einrichtung aufweist, und der zweite Teil (32) die Datenverarbeitungsvorrichtung (26) aufweist, wobei der zweite Teil (32) zum Empfang der Daten der Datenaufzeichnungsvorrichtung (34) auf Abruf bestimmt ist.

8. System nach einem der Ansprüche 1 bis 7, **dadurch gekennzeichnet, dass** die Datenverarbeitungsvorrichtung (26) der äußeren Anordnung (32) geeignet ist zur Anpassung der Steuerdaten einerseits an die vom ersten Sender (18) empfangenen Daten und andererseits an weitere den lebenden Körper, in den die implantierbare Einheit (10) implantiert ist, betreffende Informationen.

9. System nach einem der Ansprüche 1 bis 8, **dadurch gekennzeichnet, dass** eine von der Steuer- und Regelvorrichtung (24) ermittelte Bedingung mindestens eine Bedingung im Zusammenhang mit der Frequenz umfasst, mit der der Sensor (14) das Parameter des lebenden Körpers misst.

10. System nach einem der Ansprüche 1 bis 9, **dadurch gekennzeichnet, dass** eine von der Steuer- und Regelvorrichtung (24) ermittelte Bedingung mindestens eine Bedingung im Zusammenhang mit der Ausführungsperiode der Versendung der Daten bezüglich eines Parameters des lebenden Körpers durch den ersten Sender (18) umfasst.

11. System nach einem der Ansprüche 1 bis 10, **dadurch gekennzeichnet, dass** die Steuer- und Regelvorrichtung (24) der implantierbaren Einheit außerdem dazu geeignet ist, eine Datenverarbeitung vorzunehmen.

12. System nach Anspruch 11, **dadurch gekennzeichnet, dass** die durch die Steuer- und Regelvorrichtung (24) der implantierbaren Anordnung vorgenommene Datenverarbeitung eine Kompression der durch den ersten Sender (18) zu sendenden Daten umfasst.

13. System nach einem der Ansprüche 1 bis 12, **dadurch gekennzeichnet, dass** das System außerdem eine Alarmvorrichtung (38) aufweist, und dass die Steuer- und Regelvorrichtung (24) der implantierbaren Einrichtung, direkt oder anderweitig, die Alarmvorrichtung (38) auslöst, wenn eine Bedingung im Zusammenhang mit dem durch den Sensor (14) gemessenen Parameter erfüllt ist.

14. System nach Anspruch 13, **dadurch gekennzeichnet, dass** die Bedingung im Zusammenhang mit dem gemessenen Parameter die Überschreitung mindestens eines unteren oder oberen Grenzwertes des Parameters ist.
